(19) Europäisches Patentamt
European Patent Office
Office européen des brevets

(11) **EP 4 335 353 A1**

(12) **EUROPEAN PATENT APPLICATION**

(43) Date of publication:
**13.03.2024 Bulletin 2024/11**

(21) Application number: **22306322.3**

(22) Date of filing: **07.09.2022**

(51) International Patent Classification (IPC):
**A61B 3/09** *(2006.01)*    **A61B 3/08** *(2006.01)*

(52) Cooperative Patent Classification (CPC):
**A61B 3/09; A61B 3/08**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Essilor International**
**94220 Charenton-Le-Pont (FR)**

(72) Inventors:
• **POULAIN, Isabelle**
**94370 SUCY EN BRIE (FR)**

• **MARIN, Gildas**
**75012 PARIS (FR)**
• **BARRETO, Noemi**
**91450 SOISY SUR SEINE (FR)**
• **ERNOULT, Lucie**
**93100 MONTREUIL (FR)**

(74) Representative: **Ipsilon**
**Le Centralis**
**63, avenue du Général Leclerc**
**92340 Bourg-la-Reine (FR)**

(54) **A METHOD AND DEVICE FOR DETERMINING A PERSONALIZED NEAR VISION INDEX**

(57) This method for determining a personalized near vision index for an individual comprises: measuring (10) at least one parameter related to accommodation of the individual; determining (12) a binocular vision model representing vision of the individual; obtaining (14) the personalized near vision index as comprising a binocular visual effort of the individual in near vision, the binocular visual effort being evaluated as a function of the measured at least one parameter related to accommodation and of the determined binocular vision model.

```
┌─────────────────────────────────────────────────┐
│  Measuring parameter(s) related to accommodation │ ── 10
└─────────────────────────────────────────────────┘
                        │
                        ▼
┌─────────────────────────────────────────┐
│   Determining binocular vision model     │ ── 12
└─────────────────────────────────────────┘
                        │
                        ▼
┌─────────────────────────────────────────┐
│    Evaluating binocular visual effort    │ ── 14
└─────────────────────────────────────────┘
                        │
                        ▼
        Personalized near vision index
```

**FIG.1**

EP 4 335 353 A1

**Description**

FIELD OF THE INVENTION

The present invention relates to a method and device for determining a personalized near vision index.

BACKGROUND OF THE INVENTION

At an early stage of presbyopia, patients feel discomfort at near vision: they experience visual fatigue and/or near vision visual performance decrease.

However, they often do not know that such discomfort is due to their vision and do not know that there are optical correction solutions either. Even if they have heard about presbyopia, they do not feel concerned.

Besides, most patients use a workaround to compensate for near vision issues and wait, before being equipped with ophthalmic lens correction for near vision i.e. addition, until the moment they are suffering enough.

On the other hand, recent consumer studies carried out on this issue show that consumers want to be actors in managing their vision. In addition, practitioners do not know when they should start an eye examination at near vision or propose equipment for visual comfort.

In the state of the art, near vision performance of an individual can be measured, but there is no method for evaluating visual comfort at near vision of an individual who does not wear any visual equipment.

Thus, there is a need for consumers and practitioners to characterize visual comfort at the early stage of presbyopia and intake of ophthalmic lens correction in a way that is easy to understand for the wearer.

SUMMARY OF THE INVENTION

An object of the present disclosure is to overcome the above-mentioned drawbacks of the prior art.

To that end, the present disclosure provides a method for determining a personalized near vision index for an individual, remarkable in that it comprises:

measuring at least one parameter related to accommodation of the individual;
determining a binocular vision model representing vision of the individual;
obtaining the personalized near vision index as comprising a binocular visual effort of the individual in near vision, the binocular visual effort being evaluated as a function of the measured at least one parameter related to accommodation and of the determined binocular vision model.

Thus, the method according to the disclosure makes it possible to associate the personalized near vision index with recommendations for visual correction and therefore, this makes it possible, on one hand, for the consumer to be aware of presbyopia, its evolution and available optical solutions, to understand presbyopia and be actor when choosing an optical solution and on the other hand, for the Eye Care Professional (ECP) to objectify near vision in the refraction room and decide on whether or not to start an eye examination at near vision and, in a store, to help the consumer in visual need analysis and guide him or her to the best solution.

Namely, the proposed personalized near vision index is a score based on several criteria that will be described in detail hereafter, that gives an indication of where each individual stands in terms of visual comfort or effort at near vision, where he or she could be thanks to optical correction solutions and that allows to predict the evolution of visual comfort.

The main outcome of the personalized near vision index is similar to an accommodation curve like in the health journal, to position and follow near vision evolution for an individual, except that it does not only take into account pure accommodation capabilities.

In an embodiment, the at least one parameter related to accommodation is taken in a group comprising the amplitude of accommodation, the full accommodative response from far vision to near vision, the punctum proximum of accommodation, the punctum remotum of accommodation, the dark focus and the microfluctuation amplitude.

In an embodiment, the measuring further comprises measuring binocular vision of the individual, including measuring at least one parameter among phoria, fusional reserves, the accommodative convergence/accommodation ratio, the convergence accommodation/convergence ratio, other accommodation-convergence crosslinks and other convergence capabilities.

In an embodiment, the binocular vision model comprises a model of accommodation, convergence, accommodation-convergence cross-links as a function of blur and disparity.

In an embodiment, the binocular visual effort comprises a motor effort that is a brain effort made by the individual to accommodate and to converge and a process effort that is a brain effort made by the individual to compensate for the blur and for the disparity.

In an embodiment, the binocular visual effort is the sum of the motor effort and the process effort, the motor effort $E_m$ and the process effort $E_p$ being given by the following formulas for accommodation, respectively convergence:

$$E_m = k_m(R\text{-}X)^2$$

$$E_p = k_p(S\text{-}R)^2$$

where, for accommodation, respectively convergence:

$k_m$ and $k_p$ are predetermined coefficients,
S is the stimulus,
R is the total response,
X is the reflex response of the accommodation, respectively of the convergence.

In an embodiment, the individual is wearing ophthalmic correction and the method further comprises using the personalized near vision index to evaluate visual comfort of the individual with the ophthalmic correction.

In an embodiment, the method further comprises using the personalized near vision index to recommend an ophthalmic correction offering the best visual comfort to the individual.

In an embodiment, the personalized near vision index takes discrete values.

In another embodiment, the personalized near vision index is a continuous scale.

In an embodiment, the personalized near vision index further comprises a vertical disparity effort.

In an embodiment, the vertical disparity effort is given by the following formula:

$$(vdisp/maxdisparityVer)^2$$

where vdisp is the vertical disparity and maxdisparityVer is the maximum vertical disparity, which equals the vertical fusion reserve.

To the same end as mentioned above, the present disclosure also provides a device for determining a personalized near vision index for an individual, remarkable in that it comprises:

means for measuring at least one parameter related to accommodation of the individual;
means for determining a binocular vision model representing vision of the individual;
means for obtaining the personalized near vision index as comprising a binocular visual effort of the individual in near vision, the obtaining means being adapted to evaluate the binocular visual effort as a function of the measured at least one parameter related to accommodation provided by the means for measuring the at least one parameter related to accommodation and of the binocular vision model provided by the means for determining the binocular vision model.

To the same end as mentioned above, the present disclosure also provides a computer program product, remarkable in that it comprises one or more sequences of instructions that are accessible to a processor and that, when executed by the processor, cause the processor to implement a method for determining a personalized near vision index for an individual, the processor being configured for:

obtaining at least one measured parameter related to accommodation of the individual;
determining a binocular vision model representing vision of the individual;
obtaining the personalized near vision index as comprising a binocular visual effort of the individual in near vision, the binocular visual effort being evaluated as a function of the measured at least one parameter related to accommodation and of the determined binocular vision model.

To the same end as mentioned above, the present disclosure further provides a non-transitory information storage medium, remarkable in that it stores one or more sequences of instructions that are accessible to a processor and that, when executed by the processor, cause the processor to implement a method for determining a personalized near vision index for an individual, the processor being configured for:

obtaining at least one measured parameter related to accommodation of the individual;
determining a binocular vision model representing vision of the individual;
obtaining the personalized near vision index as comprising a binocular visual effort of the individual in near vision, the binocular visual effort being evaluated as a function of the measured at least one parameter related to accommodation and of the determined binocular vision model.

As particular features and advantages of the device, computer program product and information storage medium are similar to those of the method, they are not repeated here.

The computer program product is advantageously configured for executing the method in any of its execution modes.

BRIEF DESCRIPTION OF THE DRAWINGS

For a more complete understanding of the description provided herein and the advantages thereof, reference is now made to the brief descriptions below, taken in connection with the accompanying drawings and detailed description, wherein like reference numerals represent like parts.

FIG. 1 is a flow diagram showing steps of a method according to the present disclosure, in a particular embodiment.
FIG. 2 is a schematic view of an example of the modification, by an ophthalmic lens, of accommodative stimuli and of vergence stimuli used in a method according to the present disclosure, in a particular embodiment.
FIG. 3 is a graph showing the visual effort in near vision according to the needed addition when the considered individual does not wear any ophthalmic correction, the visual effort being evaluated in a method according to the present disclosure, in a particular embodiment.
FIG. 4 is a graph showing the visual effort in near vision according to the needed addition when the considered individual wears an ophthalmic correction, the visual effort being evaluated in a method according to the present disclosure, in a particular embodiment.

DETAILED DESCRIPTION OF EMBODIMENTS

In the description which follows, the drawing figures are not necessarily to scale and certain features may be shown in generalized or schematic form in the interest of clarity and conciseness or for informational purposes. In addition, although making and using various embodiments are discussed in detail below, it should be appreciated that as described herein are provided many inventive concepts that may embodied in a wide variety of contexts. Embodiments discussed herein are merely representative and do not limit the scope of the invention. It will also be obvious to one skilled in the art that all the technical features that are defined relative to a process can be transposed, individually or in combination, to a device and conversely, all the technical features relative to a device can be transposed, individually or in combination, to a process.

The terms "comprise" (and any grammatical variation thereof, such as "comprises" and "comprising"), "have" (and any grammatical variation thereof, such as "has" and "having"), "contain" (and any grammatical variation thereof, such as "contains" and "containing"), and "include" (and any grammatical variation thereof such as "includes" and "including") are open-ended linking verbs. They are used to specify the presence of stated features, integers, steps or components or groups thereof, but do not preclude the presence or addition of one or more other features, integers, steps or components or groups thereof. As a result, a method, or a step in a method, that "comprises", "has", "contains", or "includes" one or more steps or elements possesses those one or more steps or elements, but is not limited to possessing only those one or more steps or elements.

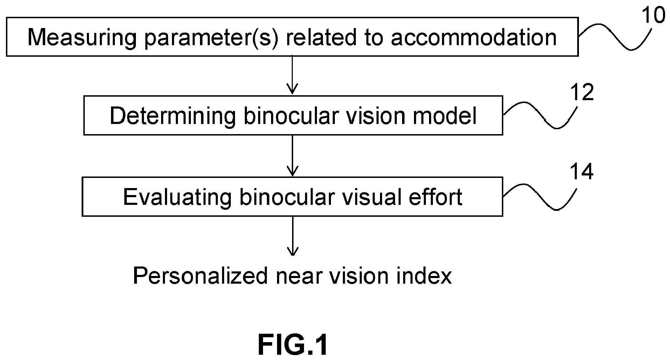

Figure 1 shows steps of a method according to the present disclosure for determining a personalized near vision index for an individual, in a particular embodiment.

A first step 10 of the method comprises measuring at least one parameter related to accommodation of the individual.

The at least one parameter related to accommodation may be one or several of the following: the amplitude of accommodation, the full accommodative response from far vision to near vision, the punctum proximum of accommodation, the punctum remotum of accommodation, the dark focus and the microfluctuation amplitude.

Optionally, at step 10, in addition to measuring at least one parameter related to accommodation, the method may further comprise measuring binocular vision of the individual. This includes measuring one or several parameters among the following: phoria, fusional reserves, the accommodative convergence/accommodation ratio, the convergence accommodation/convergence ratio, other accommodation-convergence crosslinks and other convergence capabilities.

Indeed, the amplitude of accommodation is a physiological parameter that undeniably evolves with age and is mainly used as reference to explain near vision issues. However, the reference to age for the management of near vision issues is not sufficient. To fully characterize the accommodative response, many other parameters may be assessed, such as the dark focus, also referred to as resting or tonic accommodation, the accommodative gain, characterizing the lead and lag, the crosslinks gain with convergence (the ACA ratio or accommodative convergence/accommodation ratio and the CAC ratio or convergence accommodation/convergence ratio), the proximal accommodation, etc. Moreover, the accommodative response may be influenced by binocular vision problems (phoria, fusional reserves, etc.), by the pupil diameter, etc.

As shown in Figure 1, a following step 12 comprises determining a binocular vision model, representing vision of the individual.

By way of non-limiting example, the binocular vision model may comprise a model of accommodation, convergence, accommodation-convergence cross-links as a function of blur and disparity. The proposed model preferentially tries to keep coherent known relations with age and luminance.

After step 12, a step 14 is carried out, comprising obtaining the personalized near vision index. The personalized near vision index comprises a binocular visual effort of the individual in near vision. The binocular visual effort is evaluated as a function of the at least one parameter related to accommodation measured at step 10 and of the binocular vision model determined at step 12.

The personalized near vision index may either take discrete values (e.g. two basic values "comfortable" and "not comfortable", six values ranging from "not at all comfortable" to "very comfortable", etc.), or be a continuous scale that is not necessarily linear.

By way of non-limiting example, the binocular visual effort may comprise a motor effort and a process effort. The motor effort is a brain effort made by the individual to accommodate and to converge. The process effort is a brain effort made by the individual to compensate for the blur and for the disparity.

By way of non-limiting example, the binocular visual effort may be the sum of the motor effort and the process effort. The motor effort $E_m$ and the process effort $E_p$ may be given by the following formulas for accommodation, respectively convergence:

$$E_m = k_m(R-X)^2$$

$$E_p = k_p(S-R)^2$$

where, for accommodation, respectively convergence:

$k_m$ and $k_p$ are predetermined coefficients,
S is the stimulus,
R is the total response,
X is the reflex response of the accommodation, respectively of the convergence.

In a particular embodiment, $k_m = k(1-G)$ and $k_p = kG_x$, where G is the accommodative gain, respectively the convergence gain, $G_x = G(1-dX/dR)$ where dX/dR is the derivative of X against R and k is a normalization factor.

In another particular embodiment, the binocular visual effort may be expressed differently. The motor effort may be related to the distance from the resting point (AR-dkF), where AR is the accommodative response and dkF is the dark focus. The process effort may be related to the distance from the stimulus (AS-AR), where AS is the accommodative stimulus. The accommodation-convergence response of the visual system may correspond to the minimal effort for a given stimulation. This means that the derivative of the effort model against the response should be null at the effective response of the visual system. This will imply a quadratic effort model for a linear response model.

For a monocular accommodative response AR = AG.AS + (1 -AG)dkF where AG is the accommodative gain, $E_p = k_p.(AS-AR)^2$ with typically $k_p = 1$ and $E_m = km.(AR-dkF)^2$ with typically $k_m = (1-AG)/AG$.

Then, the monocular visual effort is $([(1-AG)/AG](AR-dkF)^2 + (AS-AR)^2)/Tol\_blur$, where Tol_blur is a normalization coefficient. For instance, Tol_blur = kmax.DoF.(PPA-PRA+DoF), where kmax is a predetermined coefficient representing a sensitivity to blur depending on the individual and the vision conditions, DoF is the depth of focus or field, PPA is the punctum proximum of accommodation and PRA is the punctum remotum of accommodation.

A binocular accommodation effort model can be defined as the averaged monocular effort with a linear or quadratic ponderation d of dominance.

With a linear ponderation :

$$AS_{binocular} = ((1+d)AS_{right\ eye} + (1-d)AS_{left\ eye})/2.$$

With a quadratic ponderation :

$$AS_{binocular} = \sqrt{((1+d)AS^2_{right\,eye} + (1-d)AS^2_{left\,eye})/2}$$

d=0 if there is no dominance, d=1 if the right eye is dominant and d=-1 if the left eye is dominant. As a variant, d may take any value between -1 and +1 to model non-binary dominance, i.e. a continuous level of dominance.

The binocular model may be derived from the monocular model with the following correspondences:

AG corresponds to AG-ACA.CAC, where ACA is the accommodative convergence and CAC is the convergence accommodation;
dkF corresponds to dkF + CAC(VS-dkV), where VS is the vergence stimulus and dkV is the dark vergence;
AR corresponds to AR + VR, where VR is the vergence response.

Then, the binocular visual effort may be given by the following formula:

$$[[(1-AG)/(AG-ACA.CAC)].[AR-[dkF+CAC(VS-dkV)]]^2 + (AS-AR)^2]/Tol\_blur +$$

$$[[(1-VG)/(VG-ACA.CAC)].[VR-[dkV+ACA(AS-dkF)]]^2 + (VS-VR)^2]/Tol\_cv$$

where Tol_blur = kmax.DoF.(PPA-PRA+kmax.DoF) and Tol_cv = PaRHor.(PPC-PRC+PaRHor) are normalization coefficients, where kmax = $\Delta$fr/$\Delta$blur, where $\Delta$fr represents the fusional reserves and $\Delta$blur is the blur limit (typically kmax is approximately equal to 1.41, also depending on age and $\Delta$blur is approximately equal to 30 diopters), and where:

AG is the accommodative gain,
ACA is the accommodative convergence,
CAC is the convergence accommodation,
AR is the accommodative response (effective accommodation plan proximity),
VR is the vergence response (effective vergence plan proximity),
VG is the vergence gain,
dkF is the dark focus,
VS is the vergence stimulus (proximity of horizontal or best gaze crossing),
dkV is the dark vergence,
AS is the accommodation stimulus (object image proximity seen through lenses, i.e. the power error calculated along the gaze direction),
PRA is the punctum remotum of accommodation (objective ametropia), which is null in case the individual is emmetrope),
PPA is the punctum proximum of accommodation (PPA = ObjAA+PRA),
DoF is the depth of focus, DoF = (DoF(PRA)+DoF(PPA))/2, DoF = max{1, (0.11+0.03.age)/(0.536+0.006.age)}.ObjDoF where ObjDoF = 1.5-0.1125[Dpup(0)+Dpup(PPA-PRA)]/2 Dpup being the pupil diameter
PaRHor is the horizontal panum radius, PaRHor = 6+SF/4+3.75Ecc (in minutes of arc) where SF is the spatial frequency and Ecc is the retinal eccentricity, PaRHor in diopters is equal to the product of PaRHor in minutes of arc with $\pi$/(60.180PD) where PD is the pupillary distance in meters,
PPC is the punctum proximum of convergence,
PRC is the punctum remotum of convergence.

Typically Tol_blur = kdof.DoF and Tol_cv = kcv.PaR where PaR is the panum radius. The maximum effort is not reached at DoF, so kdof = kmax>1 for blur, while on the contrary, convergence stops and diplopia occurs as soon as the disparity goes above the panum radius at the fusional limits, meaning kcv = 1.

As for the monocular model, Tol_blur may be chosen to get a maximum effort around 1 when the maximum tolerated blur kmax.DoF is reached, or when diplopia occurs:

The accommodation should also stop when diplopia occurs, meaning that the accommodative and convergence efforts should be the same at the limits of the fusional reserves or when AS = VS = PPC, implying kmax = f(DoF, PaR, PPC) and, giving the maximum blur at diplopia:

$$Cmax = PPC-PRC+PaR = PPA-PRA+kmax.DoF$$

$$Tol\_blur = kmax.DoF.Cmax$$

$$Tol\_cv = PaR.Cmax$$

The maximum effort is obtained at a maximum accommodative error kmax.DoF, kmax>1.

A relation with kmax and Cmax can also be derived from the following equation, obtained with Effort(AS = VS = PPC) = 1:

$$(1-ACA.CAC) = [PPC-dkF+CAC(PPC-dkV)]^2(1-AG)/kmax.DoF.Cmax +$$
$$[PPC-dkV+ACA(PPC-dkF)]^2(1-VG)/PaR.Cmax$$

$$kmax = [PPC-dkF+CAC(PPC-dkV)]^2(1-AG)/DoF/\{(1-ACA.CAC).Cmax-$$
$$[PPC-dkV+ACA(PPC-dkF)]^2(1-VG)/PaR\}$$

An effort considered as comfortable is lower than 1/3 of kmax and an effort considered as uncomfortable is higher than 2/3 of kmax.

kmax may be different according to ametropia, typically higher for myopes than for hyperopes, because myopes are more effective in processing blur.

kmax may also be a function of the stimulation, not only of the stimulus condition (luminance, contrast, spatial frequencies, etc.), but also of the motivation.

Parameters such as the dark focus, the punctum proximum and the punctum remotum may be derived from specific objective measurements, or from the prescription and/or age of the individual. These parameters may be objectively measured with an auto-refractor. The dark focus may be evaluated first using the same auto-refractor, by switching off the stimulus and waiting after a resting period of at least a few seconds (ideally 30s).

The simplest way to evaluate the dark focus is to measure PPA with a standard optometric method consisting in noting the shortest distance at which a stimulus is seen blurred, then to derive the dark focus from the model, assuming PR = Rx: dkF = ObjAA/3+PR, where PR is the punctum remotum. Another simple method is to derive ObjAA and then all other parameters from age, using the model.

Accommodation-convergence model

The monocular accommodation may be expressed as follows:

$$AR = min(max(PRA, AG.(AS-dkF)+dkF, PPA)$$

If |AS-AR|>kmax.DoF, AR=dkF

The binocular accommodation convergence may be expressed as follows:

$$AR = [(AG-CAC.ACA)AS+(1-AG)(dkF+CAC(VS-dkV))]/(1-ACA.CAC)$$

$$VR = [(VG-CAC.ACA)VS+(1-VG)(dkV+ACA(AS-dkF))]/(1-ACA.CAC)$$

$$AR = min(max(PRA, AR), PPA)$$

$$VR = \min(\max(PRC, VR), PPC)$$

If $|VS-VR|>PaRHor$, $VR = dkV$ and $AR = dkF$ (or come back to a monocular rmodel)

$AS = [(1+d)AS_{right\ eye}+(1-d)AS_{left\ eye}]/2$ with $d = 0$ if there is no dominance, $1>d>0$ if the right eye is dominant and $0>d>-1$ if the left eye is dominant.

$AG = ObjAA/(ObjAA+DoF)$, where ObjAA is the monocular objective amplitude of accommodation, $ObjAA = 7.083/(1+e^{(0.2031.(age-36.2-0.6109)})$ and $PPA = ObjAA+PRA$

$ACA = (VS+dPh_{AS}-dPh_{Rx})/AS$ or $ACA = 1-0.005.age$, where $dPh_{AS}$ is the dissociated phoria, $dPh_{AS}+VS = dkV+ACA(AS-dkF)$ and $dPh_{Rx}$ is the dissociated phoria at distance, $dPh_{Rx} = dkV+ACA.(Rx-dkF)-Rx$ where Rx is the spherical equivalent that would be prescribed for far vision obtained from a subjective refraction.

$$CAC = 1/(0.5+ACA)$$

$$VG = \Delta fr/(\Delta fr+2PaRHor).(1-ACA.CAC)+ACA.CAC$$

$$dkF = ObjAA/3+PRA$$

$dkV = ACA.dkF+dPh_{PRA}$ where $dPh_{PRA}$ is the dissociated phoria for the punctum remotum of accommodation, or $dkV = ACA.dkF$ with the hypothesis that $dPh_{PRA} = 0$

$\Delta fr$ is approximately equal to 42.5 diopters.

Optionally, the personalized near vision index may further comprise a vertical disparity effort.

In this case, by way of non-limiting example:

$E_p = (vdisp-VR)^2$ where vdisp is the vertical disparity

$$E_m = (1-VG)VR^2/VG$$

The total effort is $[(1-VG)VR^2/VG+(vdisp-VR)^2]/Tol\_disp$

For a standard linear response $VR = VG.vdisp$, the total effort is:

$[(1-VG).vdisp^2]/Tol\_disp$ where $Tol\_disp = PaRVer.maxdisparityVer$, where PaRVer is the vertical panum radius and maxdisparityVer is the maximum vertical disparity, which equals the vertical fusions reserves (typically 1.5 to 3 diopters)

$$VG = (maxdisparityVer-PaRVer)/maxdisparityVer$$

$$1-VG = PaRVer/maxdisparityVer$$

Then, the vertical disparity effort may be given by the following formula:

$$(vdisp/maxdisparityVer)^2$$

In the case where the individual is wearing ophthalmic correction or has to choose new ophthalmic lenses, the method according to the present disclosure may further comprise using the personalized near vision index obtained at step 14 to evaluate visual comfort of the individual with the ophthalmic correction.

In any case, the method according to the present disclosure may further comprise using the personalized near vision index to recommend an ophthalmic correction offering the best visual comfort to the individual.

The accommodation stimulus AS and the vergence stimulus VS are modified by a lens. Indeed, for a particular stimulus position, each lens in front of each eye has a particular power error $P_{right\ eye}$ and $P_{left\ eye}$ at the point of gazing, the power errors given by the lens power map modifying AS in ASlens as follows:

$$ASlens_{right\ eye} = AS + P_{right\ eye}$$

$$ASlens_{left\ eye} = AS + P_{left\ eye}$$

ASlens = [(1+d)ASlens$_{right\ eye}$+(1-d)ASlens$_{left\ eye}$]/2 where d=0 if there is no dominance, d=1 if the right eye is dominant and d=-1 of the left eye is dominant

The power error P is S+C/2, where S is the sphere and C is the cylinder.

Each lens in front of each eye also has a particular prismatic deviation denoted dp$_{right\ eye}$ and dp$_{left\ eye}$, modifying VS in VSlens as follows:

$$VSlens = VS + dp_{right\ eye} - dp_{left\ eye}$$

The above formulae are an approximation in the thin lens theory. For an exact result, ray tracing through the lens should be used to calculate ASlens and VSlens.

The distribution of the lens power and the prismatic deviation depends on the lens design for given stimuli positions. Thus, it is possible to assess the level of effort required for different lens designs and to select the design(s) providing a reduced effort. Therefore, the present disclosure makes it possible to predict the level of comfort for different lens designs and to help the consumer in selecting the most appropriate lens design.

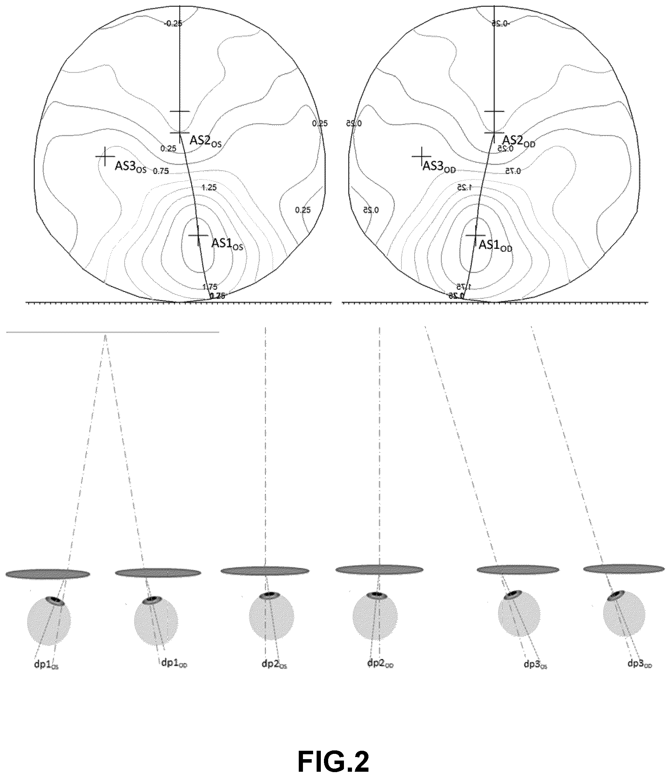

Figure 2 shows a non-limiting example of the modification of AS and VS by a lens.

A given physical set of stimuli i = 1, ..., n (n = 3) is considered. The accommodative stimuli ASi are shown for the right eye (referred to as "OD" in the drawing) and for the left eye (referred to as "OS"). The first stimulus corresponds to near vision, the second stimulus corresponds to the fitting cross and the third stimulus corresponds to a non particular gaze direction somewhere in the right field of view between far and intermediate vision.

The prismatic deviations dpi are also shown for OD and OS. Then, a set of possible lens designs may be compared to each other.

The effort can be calculated for any visual demand. Even if the most relevant efforts are in near vision, it may also be relevant to mix near vision, intermediate vision and far vision, or even different gaze directions, either in the vertical direction, (thus corresponding to an ergorama of the visual index), or in any direction (corresponding to a visual index map of the lenses).

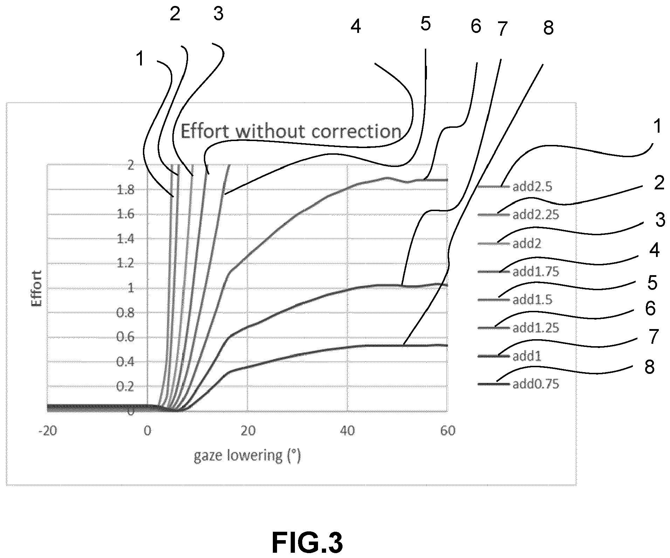

Figure 3 shows the evaluated visual effort in near vision for various gaze directions assuming a relation between gaze and distance, as a function of the needed addition given by the ergorama, the individual not wearing any correction. The abscissa axis indicates the gaze lowering in degrees. As is well known by the skilled person, an ergorama gives a relation between gaze height/lowering and the distance of objects seen. The lower the gaze, the closer the object.

It can be seen that for an addition above 1.5, the effort is unbearable (higher than 1) at near vision without any correction.

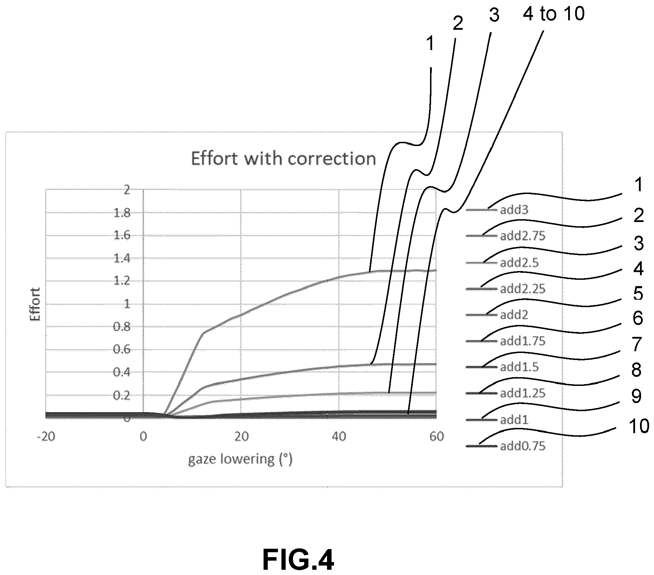

Figure 4 shows the evaluated visual effort in near vision for various gaze directions assuming a relation between gaze and distance, as a function of the needed addition given by the ergorama, the individual wearing a correction. The abscissa axis indicates the gaze lowering in degrees.

It can be seen that for an addition above 3, the effort becomes large, because the correction for low gaze is not adapted to the increasingly demanding ergorama.

A device according to the present disclosure, for determining a personalized near vision index for an individual, comprises means for measuring the above-described at least one parameter related to accommodation of the individual, means for determining a binocular vision model representing vision of the individual as described above and means for obtaining the personalized near vision index as detailed above, as comprising a binocular visual effort of the individual in near vision, the obtaining means being adapted to evaluate the binocular visual effort as a function of the measured at least one parameter related to accommodation provided by the means for measuring the at least one parameter related to accommodation and of the binocular vision model provided by the means for determining the binocular vision model.

The means for measuring the at least one parameter related to accommodation of the individual may comprise means for evaluating the amplitude of accommodation. By way of non-limiting example, such means may comprise an auto-refractor.

In another example, the means for measuring the at least one parameter related to accommodation, the means for determining the binocular vision model and the means for obtaining the personalized near vision index may be combined in a smartphone, or in a Head-mounted Virtual Reality (HVR) device.

In a particular embodiment, the method according to the disclosure is computer-implemented. Namely, a computer program product comprises one or more sequences of instructions that are accessible to a processor and that, when executed by the processor, cause the processor to carry out steps of the method for determining a personalized near vision index for an individual. The processor is configured for carrying out steps 10, 12 and 14 as described above.

The software may be hosted for example remotely in a cloud, or locally in a computer.

The sequence(s) of instructions may be stored in one or several non-transitory computer-readable storage medium/media, including a predetermined location in a cloud.

Although representative methods and devices have been described in detail herein, those skilled in the art will recognize that various substitutions and modifications may be made without departing from the scope of what is described and defined by the appended claims.

**Claims**

1. A method for determining a personalized near vision index for an individual, **characterized in that** it comprises:

   measuring at least one parameter related to accommodation of said individual;
   determining a binocular vision model representing vision of said individual;
   obtaining said personalized near vision index as comprising a binocular visual effort of said individual in near vision, said binocular visual effort being evaluated as a function of said measured at least one parameter related to accommodation and of said determined binocular vision model.

2. A method according to claim 1, **characterized in that** said at least one parameter related to accommodation is taken in a group comprising the amplitude of accommodation, the full accommodative response from far vision to near vision, the punctum proximum of accommodation, the punctum remotum of accommodation, the dark focus and the microfluctuation amplitude.

3. A method according to claim 1 or 2, **characterized in that** said measuring further comprises measuring binocular vision of said individual, including measuring at least one parameter among phoria, fusional reserves, the accommodative convergence/accommodation ratio, the convergence accommodation/convergence ratio, other accommodation-convergence crosslinks and other convergence capabilities.

4. A method according to claim 1, 2 or 3, **characterized in that** said binocular vision model comprises a model of accommodation, convergence, accommodation-convergence cross-links as a function of blur and disparity.

5. A method according to any of the preceding claims, **characterized in that** said binocular visual effort comprises a motor effort that is a brain effort made by said individual to accommodate and to converge and a process effort that is a brain effort made by said individual to compensate for the blur and for the disparity.

6. A method according to claim 5, **characterized in that** said binocular visual effort is the sum of said motor effort and said process effort, said motor effort $E_m$ and said process effort $E_p$ being given by the following formulas for accommodation, respectively convergence:

$$E_m = k_m(R-X)^2$$

$$E_p = k_p(S-R)^2$$

where, for accommodation, respectively convergence:

   $k_m$ and $k_p$ are predetermined coefficients,
   S is the stimulus,
   R is the total response,
   X is the reflex response of the accommodation, respectively of the convergence.

7. A method according to any of the preceding claims, **characterized in that** said individual is wearing ophthalmic correction and said method further comprises using said personalized near vision index to evaluate visual comfort of said individual with said ophthalmic correction.

8. A method according to any of the preceding claims, **characterized in that** it further comprises using said personalized near vision index to recommend an ophthalmic correction offering the best visual comfort to said individual.

9. A method according to any of the preceding claims, **characterized in that** said personalized near vision index takes discrete values.

10. A method according to any of claims 1 to 8, **characterized in that** said personalized near vision index is a continuous scale.

11. A method according to any of the preceding claims, **characterized in that** said personalized near vision index further comprises a vertical disparity effort.

12. A method according to claim 11, **characterized in that** said vertical disparity effort is given by the following formula:

$$(vdisp/maxdisparityVer)^2$$

where vdisp is the vertical disparity and maxdisparityVer is the maximum vertical disparity, which equals the vertical fusion reserve.

13. A device for determining a personalized near vision index for an individual, **characterized in that** it comprises:

    means for measuring at least one parameter related to accommodation of said individual;
    means for determining a binocular vision model representing vision of said individual;
    means for obtaining said personalized near vision index as comprising a binocular visual effort of said individual in near vision, said obtaining means being adapted to evaluate said binocular visual effort as a function of said measured at least one parameter related to accommodation provided by said means for measuring said at least one parameter related to accommodation and of said binocular vision model provided by said means for determining said binocular vision model.

14. A computer program product, **characterized in that** it comprises one or more sequences of instructions that are accessible to a processor and that, when executed by said processor, cause said processor to implement a method for determining a personalized near vision index for an individual, said processor being configured for:

    obtaining at least one measured parameter related to accommodation of said individual;
    determining a binocular vision model representing vision of said individual;
    obtaining said personalized near vision index as comprising a binocular visual effort of said individual in near vision, said binocular visual effort being evaluated as a function of said measured at least one parameter related to accommodation and of said determined binocular vision model.

15. A non-transitory information storage medium, **characterized in that** it stores one or more sequences of instructions that are accessible to a processor and that, when executed by said processor, cause said processor to implement a method for determining a personalized near vision index for an individual, said processor being configured for:

    obtaining at least one measured parameter related to accommodation of said individual;
    determining a binocular vision model representing vision of said individual;
    obtaining said personalized near vision index as comprising a binocular visual effort of said individual in near vision, said binocular visual effort being evaluated as a function of said measured at least one parameter related to accommodation and of said determined binocular vision model.

Measuring parameter(s) related to accommodation ⟩ 10

Determining binocular vision model ⟩ 12

Evaluating binocular visual effort ⟩ 14

Personalized near vision index

**FIG.1**

FIG.2

**FIG.3**

FIG.4

Europäisches
Patentamt
European
Patent Office
Office européen
des brevets

# EUROPEAN SEARCH REPORT

Application Number

EP 22 30 6322

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/085173 A1 (KRALL JEFFREY P [US] ET AL) 25 March 2021 (2021-03-25) | 1,13-15 | INV. A61B3/09 A61B3/08 |
| Y | * abstract *<br>* claims 1-23; figures 5-16 *<br>* paragraph [0056] – paragraph [0101] * | 2-12 | |
| Y | US 2012/019775 A1 (TYRIN ALBERT [RU] ET AL) 26 January 2012 (2012-01-26)<br>* abstract *<br>* paragraph [0054] – paragraph [0162] * | 2-12 | |
| Y | US 2021/290053 A1 (TRAN TUAN [US] ET AL) 23 September 2021 (2021-09-23)<br>* abstract *<br>* paragraph [0056] – paragraph [0218] * | 2-12 | |

TECHNICAL FIELDS
SEARCHED     (IPC)

A61B

The present search report has been drawn up for all claims

| Place of search | Date of completion of the search | Examiner |
|---|---|---|
| Munich | 27 January 2023 | Tommaseo, Giovanni |

EPO FORM 1503 03.82 (P04C01)

## ANNEX TO THE EUROPEAN SEARCH REPORT
## ON EUROPEAN PATENT APPLICATION NO.

EP 22 30 6322

This annex lists the patent family members relating to the patent documents cited in the above-mentioned European search report.
The members are as contained in the European Patent Office EDP file on
The European Patent Office is in no way liable for these particulars which are merely given for the purpose of information.

27-01-2023

| Patent document cited in search report | | Publication date | Patent family member(s) | | Publication date |
|---|---|---|---|---|---|
| US 2021085173 | A1 | 25-03-2021 | NONE | | |
| US 2012019775 | A1 | 26-01-2012 | EP | 2596402 A1 | 29-05-2013 |
| | | | RU | 2481606 C1 | 10-05-2013 |
| | | | US | 2012019775 A1 | 26-01-2012 |
| | | | WO | 2012011837 A1 | 26-01-2012 |
| US 2021290053 | A1 | 23-09-2021 | US | 2021290053 A1 | 23-09-2021 |
| | | | WO | 2021188584 A1 | 23-09-2021 |

EPO FORM P0459

For more details about this annex : see Official Journal of the European Patent Office, No. 12/82